(19) Europäisches Patentamt
European Patent Office
Office européen des brevets

(11) Publication number: **0 131 419**
**B1**

(12) # EUROPEAN PATENT SPECIFICATION

(45) Date of publication of patent specification: **04.03.87**

(21) Application number: **84304483.5**

(22) Date of filing: **29.06.84**

(51) Int. Cl.[4]: **C 07 C 103/50,**
C 07 C 101/18, C 08 K 5/17,
C 08 K 5/20, C 08 L 27/12,
C 09 D 3/78

(54) **Curing agent and fluorocarbon polymer coating composition containing curing agent.**

(30) Priority: **30.06.83 US 509705**
**30.12.83 US 566996**
**30.06.83 US 509703**
**27.02.84 US 583976**
**27.02.84 US 583977**
**30.06.83 US 509706**
**21.03.84 US 591968**
**21.03.84 US 591969**

(43) Date of publication of application:
**16.01.85 Bulletin 85/03**

(45) Publication of the grant of the patent:
**04.03.87 Bulletin 87/10**

(84) Designated Contracting States:
**BE DE FR GB NL SE**

(56) References cited:
**GB-A-1 161 410**

(73) Proprietor: **E.I. DU PONT DE NEMOURS AND COMPANY**
**1007 Market Street**
**Wilmington Delaware 19898 (US)**

(72) Inventor: **Vasta, Joseph Anthony**
**1412 Jan Drive**
**Webster Farms Wilmington, Delaware 19803 (US)**

(74) Representative: **Woodcraft, David Charles et al**
**BROOKES & MARTIN High Holborn House**
**52/54 High Holborn**
**London, WC1V 6SE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 131 419 B1

## Description

Background of the Invention

This invention is related a curing agent and in particular to a curing agent for a fluorocarbon polymer coating composition and to fluorocarbon polymer coating composition containing the curing agent.

Fluorocarbon polymers are inert to strong acids such as sulfuric acid, nitric acid, hydrochloric acid and strong bases such as sodium hydroxide and are resistant to weathering and salt water corrosion and are tough and abrasion resistant. Coatings of these polymers would be useful in chemical plants and oil refineries to coat pipes, vessels and other equipment, on off shore oil well platforms, on ships, and as protective coatings for the interior of smoke stacks of utility companies. Fluorocarbon polymer coatings would be particularly useful for metal smoke stack interiors which are subjected to abrasion from fly ash and corrosion by acids resulting from combustion products such as $SO_x$ and $NO_x$ and halogen ions. However, conventional fluorocarbon polymer coatins require curing at elevated temperatures which could not be used on the aforementioned structures. An ambient curing fluorocarbon polymer coating composition is required.

The curing agent of this invention is used in fluorocarbon polymer coating composition and provides novel compositions that cure at ambient temperatures.

Summary of the Invention

A curing agent for fluorocarbon polymer coating compositions selected from the following group:

(1)

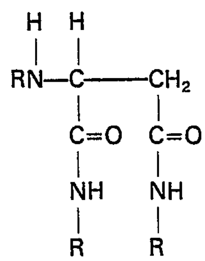

(2)

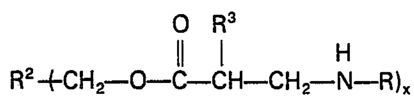

where R is $R^1NH_2$, $R^1$ is an aliphatic or cycloaliphatic hydrocarbon radical, $R^2$ is C or a hydrocabon group, $R^3$ is H or $CH_3$; where x is 3 when $R^2$ is a hydrocarbon group and x is 4 when $R^2$ is C.

Fluorocarbon polymer coating compositions containing the above curing agent also are part of the invention.

Detailed Description of the Invention

Curing agent (1) is prepared by reacting 1 mole of dialkyl maleate with 3 moles of an aliphatic or cycloaliphatic polyamine at about 100—150°C for about 1—6 hours. Alkanol formed in the reaction is removed. Preferred reaction conditions are about 110—130°C for about 2—4 hours. Preferably, the above constituents are in an alkanol solvent such as methanol, propanol isopropanol and the like.

Typical dialkyl maleates that can be used to form the curing agent are dimethyl maleate, diethyl maleate, dipropyl maleate, diisopropyl maleate, methyl ethyl maleate, methyl propyl maleate, ethyl propyl maleate, dibutyl maleate and the like. Dimethyl maleate is preferred.

Typical polyamines used to form the curing agent are isophorone diamine which is 3-aminomethyl-3,5,5-trimethylcyclohexylamine, hexamethylene diamine, ethylene diamine, 1,4-cyclohexane bis(methylamine), 1,2-diaminopropane, propylene diamine, diethyl ether diamine, trimethylhexamethyl methylene diamine.

One preferred curing agent is the reaction product of isophorone diamine and dimethyl maleate and has the following structural formula:

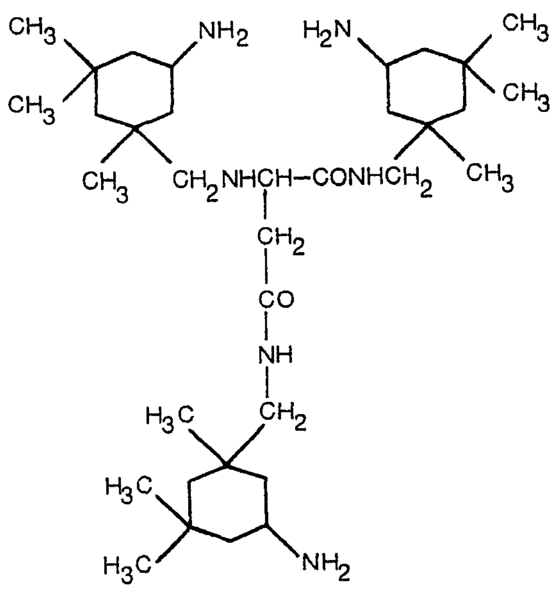

Curing agent (2) is prepared through a Michael's reaction in which a multifunctional acrylate or methacrylate is reacted with a polyamine. The polyamine is heated to about 100—150°C and then the multifunctional acrylate or methacrylate is reacted with the polyamine for a 1—6 hour period to form an amine terminated curing agent.

Typical multifunctional acrylates or methacrylates that can be used to form curing agent (2) are trimethylol propane acrylate, trimethylol propane methacrylate, pentaerythritol acrylate, pentaerythritol methacrylate and the like. Any of the aforementioned polyamines can be used to form the curing agent (2).

The curing agents are used in fluorocarbon polymer coating compositions or primer compositions. Generally, about 0.5—25% by weight, based on the weight of the binder of the composition, of curing agent is used.

One of the advantages of compositions that contain the curing agent is that these compositions cure at ambient temperatures and baking is not required. Therefore, the compositions can be used on large structures such as chemical storage tanks, chemical reactors, the interior of smoke stacks and the like which could not be subjected to baking temperatures using conventional techniques.

Typical coating composition contains about 10—70% by weight binder and 30—90% by weight of an organic solvent, in which the binder is

a fluorocarbon polymer of vinylidene fluoride and hexafluoropropylene and has a weight average molecular weight of about 50,000,600,000 and

a metallic oxide such as magnesium oxide which is an acid acceptor and the curing agent.

Usually the composition contains a reinforcing pigment such as titanium dioxide or carbon black.

The fluorocarbon polymer used in the coating composition contains about 50—70% by weight of vinylidene fluoride and 30—50% by weight of hexafluoropropylene. The polymer can contain up to 40% by weight of other monomers such as tetrafluoroethylene. One useful polymer contains about 20—30% by weight of tetrafluoroethylene.

Preferably, fluorocarbon polymers are used that have a weight average molecular weight of about 75,000—450,000. Fluorocarbon polymers having a weight average molecular weight of 50,000—300,000 are useful. Two particularly useful fluorocarbon polymers have weight average molecular weight of about 75,000—125,000 and 150,000—250,000. Polymers in the lower end of the molecular weight range are preferred for forming a composition with higher binder content. Fluorocarbon polymers in the higher molecular weight range of 300,000—450,000 are also very useful for forming coating compositions.

Molecular weight, as used herein, is determined by gel permeation chromatography using polymethyl methacrylate as a standard.

A metallic oxide which is an acid acceptor is used in the composition to react with the hydrofluoric acid which is generated during the curing or crosslinking reaction. Typical metallic oxides are magnesium oxide, lead oxide, calcium oxide, lead hydrogen phosphite and a mixture of calcium oxide and magnesium oxide. Magnesium oxide is preferred. Highly purified magnesium oxide is particularly preferred for high quality finishes.

Generally, the binder of a coating composition contains about 55—90% by weight, of the fluorocarbon polymer, 0.5—25% by weight of one of the above amine curing agents and 5—20% by weight of a metallic oxide which is an acid acceptor.

The coating composition also can contain dispersed fluorocarbon polymers such as polytetrafluoroethylene, fluorinated ethylene/propylene polymers, polyvinyl fluoride, polyvinylidene fluoride, copolymer of tetrafluoroethylene/perfluoroalkoxy vinyl ether and the like. These dispersed fluorocarbon polymers are present in amounts of about 5—20% by weight, based on the weight of the binder of the composition and have a particle size of about 1—150 microns.

A soluble low molecular weight fluorocarbon polymer of vinylidene fluoride and hexafluoropropylene having a weight average molecular weight of about 5,000—15,000 can be used in combination with a higher molecular weight fluorocarbon polymer of vinylidene fluoride and hexafluoropropylene. This low molecular weight polymer aids in dispersing pigments and the dispersed fluorocarbon polymer and forms films of 1000 microns and above without cracking of the coating on curing.

Coating compositions that contain the above low molecular weight fluorocarbon polymer have a binder containing about 50—84.5% by weight, of the solution fluorocarbon polymer, 5—20% by weight of a low molecular weight fluorocarbon polymer, 5—20% by weight of dispersed fluorocarbon polymer, 0.5—20% by weight of one of the above amine curing agents and 5—20% by weight of a metallic oxide which is an acid acceptor such as magnesium oxide.

Typical organic solvents that are used in the coating composition are acetone, tetrahydrofuran, methyl ethyl ketone, ethyl acetate, propyl acetate, butyl acetate, isobutyl acetate, methyl isobutyl ketone, methyl amyl acetate, diisobutyl ketone, ethylene glycol monomethyl ether acetate, ethylene glycol monoethyl ether and mixtures of the above. These solvents are used to make the compositions and can be used to reduce the compositions to an application viscosity.

Preferably, the coating composition contains a reinforcing agent such as titanium dioxide pigment usually in a pigment to binder weight ratio of about 20:100 to 200:100. Other inert pigments can be used such as barytes, barium sulfate, fibrous calcium silicate and the like. Carbon black, bone black or lamp black can also be used as a reinforcing pigment in a pigment to binder weight ratio of about 20:100 to 50:100.

To decrease curing time and increase toughness of the resulting finish of the coating composition about 0.01—3% by weight based on the weight of the binder, of a bicyclic amidine can be added. One preferred bicyclyic amidine is 1,8-diaza-bicyclo(5,4,0)undecene-7.

To improve resistance to acids at ambient temperatures and elevated temperatures of finishes formed by the composition about 1—20% by weight, based on the weight of the binder, of a perfluoroalkylpropylene oxide can be added. The perfluoroalkylpropylene oxide has the formula

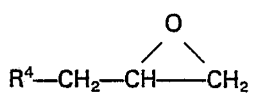

where $R^4$ is a perfluoroalkyl group having 4—12 carbon atoms. Preferably, $R^4$ is a perfluoroalkyl group of a mixture of 6—10 carbon atoms. Preferably, about 5—15% by weight based on the weight of the binder, of the perfluoroalkylpropylene oxide is added.

Other advantages of the perfluoroalkylpropylene oxide are as follows: the surface tension of the coating is lowered and the resulting finish has improved release characteristics in comparison to finishes that do not contain the perfluoroalkylpropylene oxide; the wetting of pigments is improved and makes dispersion of pigments less difficult and fluorocarbon polymer solids of the composition can be increased.

A combination of the aforementioned bicyclic amidine and the perfluoroalkyl propylene oxide allows the removal of the metallic oxide from the coating composition and still provides a composition that cures at ambient temperatures. Generally, about 1—10% by weight, based on the weight of binder, of the combination is used. The weight ratio of bicyclic amidine to perfluoroalkylpropylene oxide is about 1:1 to 1:5.

To improve flow of the coating composition and enhance smoothness of the resulting finish about 1—20% by weight, based on the weight of binder, of a glycidyl ester can be added to the composition. Preferably, about 5—15% by weight of glycidyl ester is used. The ester has the formula

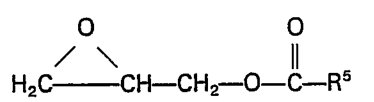

where $R^5$ is a tertiary aliphatic hydrocarbon group having 8—10 carbon atoms.

Generally, the coating composition is sold in two components. The solvents, fluorocarbon polymer, pigments such as titanium dioxide and metallic oxide acid acceptor are the first component and the amine curing agent is the second component. The second component is blended with the first component by the user to form a coating composition. The composition is then reduced with one of the aforementioned solvents to an application viscosity and then applied to a substrate.

The coating composition can be applied directly over a wide variety of substrates and provide a fluorocarbon polymer coating. Typical substrates are treated or primed steel, phosphatized steel, grit blasted steel, galvanized steel, aluminum, copper, brass, cement and cementitious surfaces such as fire brick, mortar used for fire brick and the like.

The coating composition is sprayed applied to the substrate and the solvent is allowed to flash off between coatings then the resulting coating composition is cured at ambient temperatures. The coating can be cured in about 4 to 48 hours or longer or can be heated to 80°C to 120°C for 0.5 to 2.0 hours for rapid curing. Cured coatings are about 75—1500 microns thick.

For some substrates such as untreated steel, a fluorocarbon primer is first applied and then while the primer is still wet the coating composition is applied and dried at ambient temperatures or at the above elevated temperatures.

One useful primer contains the aforementioned fluorocarbon polymer of hexafluoropropylene and vinylidene fluoride, a metallic oxide acid acceptor such as magnesium oxide and an aminoalkyly-alkoxy silane such as amino-propyl trimethoxysilane or amino-propyl triethoxysilane.

The ambient curing characteristics of the coating composition provided by the novel curing agent of this invention allows for the application of coatings on large vessels and reactors in chemical plants, and oil refineries, large structures and equipment and pipes, heat risers, i.e., pipes which are used to transport oil from the underground well to the structure, off shore oil well platforms, and on the interior of smoke stacks used by large utility companies. It is practical to use compositions that cure at ambient temperature for the above applications.

The following examples illustrate the invention. All parts and percentages are on a weight basis unless otherwise indicated.

## Example 1

A curing agent was prepared by charging the following constituents into a reaction vessel equipped with a stirrer, a heating mantel and a condenser:

| | Parts by Weight |
|---|---|
| Portion 1 | |
| Isophorone diamine | 535.5 |
| Portion 2 | |
| Dimethyl maleate | 144.0 |
| Portion 3 | |
| Isopropanol | 516.0 |
| Total | 1195.5 |

Portion 1 was charged into the reaction vessel and heated to about 120°C and then portion 2 was added at a uniform rate over a 4 hour period while the temperature was held at 130°C and methanol was removed from the vessel. After the 4 hour period heating was stopped and a sample was removed and tested in an infrared spectrophotometer which showed the reaction was complete. Then portion 3 was added to give a 55% solids solution.

About 5 parts of the above curing agent was added to about 300 parts of each of the following coating compositions:

Coating Composition A

Fluorocarbon polymer solution containing 33% solids in ethyl acetate, of a copolymer of 40% hexafluoropropylene and 60% vinylidene fluoride having a weight average molecular weight of about 100,000 and about 5% by weight magnesium oxide.

Coating Composition B

Fluorocarbon polymer solution identical to composition A except the copolymer has a molecular weight of about 200,000.

Coating Composition C

Fluorocarbon polymer solution identical to composition A except the composition contains additionally about 25% by weight of finely divided polyvinylidene fluoride.

Each composition was diluted with methyl ethyl ketone to a spray viscosity and sprayed onto aluminum and steel panels to form a coating having a dry film thickness of about 1000 microns. After drying under ambient temperature conditions for 7 days, each of the coatings is resistant to sulfuric acid, sodium hydroxide, steam and methyl ethyl ketone which indicates that the coatings were fully cured and crosslinked.

Example 2

The following amine curing agents were prepared:

| | CURING AGENT | | | |
|---|---|---|---|---|
| | Parts by Weight | | | |
| | D | E | F | G |
| Isophorone diamine | 510 | — | 562 | — |
| Hexamethylene diamine | — | 321 | — | 388 |
| Trimethylol propane aerylate | 296 | 296 | — | — |
| Pentaerythritol acrylate | — | — | 298 | 298 |
| Isopropanol | 659 | 505 | 703 | 562 |
| Total | 1465 | 1122 | 1563 | 1248 |

In the preparation of each of the above curing agents D—G, the amine was charged into a reaction vessel and heated to 120—140°C and then the acrylate was slowly added at a uniform rate over a 4 hour period and then the reaction mixture was cooled and isopropanol added.

Trimethylol propane acrylate and pentaerythritol acrylate were prepared by conventional techniques well known to the skilled in the art in which an acrylate moiety was attached to trimethylol propane and with pentaerythritol.

Separate coating compositions were prepared with each of the curing agents D—G using the same constituents as in Example 1 for the coating composition A except the above curing agent was substituted for the curing agent of Example 1. In each case the resulting coating composition was reduced to a spray viscosity with methyl ethyl ketone as in Example 1 and sprayed onto grit blasted steel panels allowing the coating to flash dry between each application to provide a 1000 micron thick dry coating. After 7 days, the coatings were fully cured but were softer and more elastic than the coating of Example 1. The coatings were resistant to sulfuric acid, sodium hydroxide and methyl ethyl ketone.

Example 3

A coating composition was prepared as follows:

| Portion 1 | Parts by Weight |
|---|---|
| Fluorocarbon polymer solution (33.3% solids in ethyl acetate of a copolymer of 60% vinylidene fluoride and 40% hexafluoropropylene having a weight average molecular weight of about 400,000) | 1744.15 |
| Barytes pigment | 580.79 |
| Carbon black pigment | 0.60 |
| Magnesium oxide | 87.19 |
| Perfluoroalkylpropylene oxide of the formula 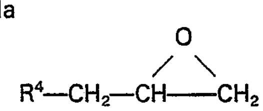 $R^4$ is a mixture of perfluoroalkyl group of 1% $C_4$, 48.5% $C_6$, 34.0% $C_8$, 12.0% $C_{10}$, 2.5% $C_{12}$ and 2% $C_{14}$. | 29.06 |
| Butyl acetate | 1158.21 |

Portion 2

| | |
|---|---|
| Amine curing agent solution (prepared in Example 1) | 49.15 |
| Total | 3649.15 |

The constituents of portion 1 were charged into a mixing vessel and blended and charged into a ball mill and ground to thoroughly disperse the pigments.

Portion 2 was added and blended. The resulting composition was reduced with methyl ethyl ketone solvent to a spray viscosity of about 25 seconds using a No. 2 Zahn cup and sprayed onto grit blasted steel panels and aluminum panels. In each case, thick films were applied by allowing flash drying between each application and the coatings were dried at ambient temperature in 24 hours to a tack free condition to provide a 1000 micron thick dry coating. In each case, the coating had excellent adhesion to the metal substrate. After 7 days, the coatings were fully cured and were resistant to sulfuric acid, sodium hydroxide and solvents such as methyl ethyl ketone and ethyl acetate.

Example 4

A pigment dispersion was prepared as follows:

| | Parts by Weight |
|---|---|
| Fluorocarbon polymer solution (described in Example 3) | 1890.0 |
| Titanium dioxide pigment | 630.0 |
| Butyl acetate | 1080.0 |
| Total | 3600.0 |

The above constituents were charged into a ball mill and ground to thoroughly disperse the pigment. A coating composition was prepared by thoroughly blending together the following constituents:

| | Parts by Weight |
|---|---|
| Pigment dispersion (prepared above) | 971.4 |
| Perfluoroalkylpropylene oxide (described in Example 3) | 30.0 |
| Amine curing agent solution (prepared in Example 1) | 6.0 |
| Bicyclic amidine solution (10% solids in butyl acetate of 1,8-diaza bicyclo-(5,4,0)-undecene-7) | 2.0 |
| Total | 1009.4 |

The resulting composition was reduced with methyl ethyl ketone solvent to a spray viscosity of about 37 seconds using a No. 2 Zahn cup and sprayed onto grit blasted steel panels and aluminum panels. In each case, thick films were applied by allowing flash drying between each application and the coatings were dried at ambient temperature in 24 hours to a tack free condition to provide a 1000 micron thick dry coating. In each case, the coating had excellent adhesion to the metal substrate. After 7 days, the coatings were fully cured and were resistant to sulfuric acid, sodium hydroxide and solvents such as methyl ethyl ketone and ethyl acetate.

Example 5

A pigment dispersion was prepared as follows:

| | | Parts by Weight |
|---|---|---|
| Fluorocarbon polymer solution (described in Example 3) | | 1890.0 |
| Titanium dioxide pigment | | 630.0 |
| Magnesium oxide | | 31.5 |
| Butyl acetate | | 1138.5 |
| | Total | 3690.0 |

The above constituents were charged into a ball mill and ground to thoroughly disperse the pigment.

A coating composition was prepared by thoroughly blending together the following constituents:

| | | Parts by Weight |
|---|---|---|
| Pigment dispersion (prepared above) | | 527.2 |
| Perfluoroalkylpropylene oxide (described in Example 3) | | 10.0 |
| Amine curing agent solution (prepared in Example 1) | | 3.0 |
| Bicyclic amidine solution (described in Example 4) | | 1.0 |
| | Total | 541.2 |

The resulting composition was reduced with methyl ethyl ketone solvent to a spray viscosity of about 37 seconds using a No. 2 Zahn cup and sprayed onto grit blasted steel panels and aluminum panels. In each case, thick films were applied by allowing flash drying between each application and the coatings were dried at ambient temperature in 24 hours to a tack free condition to provide a 1000 micron thick dry coating. In each case, the coating had excellent adhesion to the metal substrate. After 7 days, the coatings were fully cured and were resistant to sulfuric acid, sodium hydroxide and solvents such as methyl ethyl ketone and ethyl acetate.

Example 6

A coating composition was prepared by thoroughly blending together the following constituents:

| | | Parts by Weight |
|---|---|---|
| Pigment dispersion (prepared in Example 5) | | 527.2 |
| "Cardura" E Ester (glycidyl ester of the formula 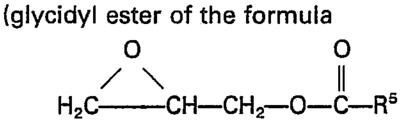 where $R^5$ is a tertiary aliphatic hydrocarbon group of 8—10 carbon atoms) | | 10.0 |
| Amine curing agent solution (prepared in Example 1) | | 3.0 |
| Bicyclic amidine solution (described in Example 4) | | 1.0 |
| | Total | 541.2 |

The resulting composition was reduced with methyl ethyl ketone solvent to a spray viscosity of about 37 seconds using a No. 2 Zahn cup and sprayed onto grit blasted steel panels and aluminum panels. In each case, thick films were applied by allowing flash drying between each application and the coatings were dried at ambient temperature in 24 hours to a tack free condition to provide a 1000 micron thick dry coating. In each case, the coating was very smooth and had excellent adhesion to the metal substrate. After 7 days, the coatings were fully cured and were resistant to sulfuric acid, sodium hydroxide and solvents such as methyl ethyl ketone and ethyl acetate.

Example 7

A coating composition was prepared by blending together the following constituents:

| | Parts by Weight |
|---|---|
| Pigment Dispersion (prepared in Example 5) | 527.2 |
| "Cardura" E Ester (described in Example 6) | 5.0 |
| Perfluoroalkylpropylene oxide (described in Example 3) | 5.0 |
| Amine curing agent solution (prepared in Example 1) | 3.0 |
| Bicyclic amidine solution (described in Example 4) | 1.0 |
| Total | 541.2 |

The resulting composition was reduced with methyl ethyl ketone solvent to a spray viscosity of about 37 seconds using a No. 2 Zahn cup and sprayed onto grit blasted steel panels and aluminum panels. In each case, thick films were applied by allowing flash drying between each application and the coatings were dried at ambient temperature in 24 hours to a tack free condition to provide a 1000 micron thick dry coating. In each case, the coating was very smooth and had excellent adhesion to the metal substrate. After 7 days, the coatings were fully cured and were resistant to sulfuric acid, sodium hydroxide and solvents such as methyl ethyl ketone and ethyl acetate.

Example 8

A coating composition was prepared as follows:

| Portion 1 | Parts by Weight |
|---|---|
| Fluorocarbon polymer solution (33.3% solids in ethyl acetate of a copolymer of 60% vinylidene fluoride and 40% hexafluoropropylene having a weight average molecular weight of about 400,000) | 638.12 |
| Perfluoroalkyl propylene oxide of the formula 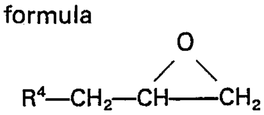 $R^4$ is a mixture of perfluoroalkyl group of 1% $C_4$, 48.5% $C_6$, 34.0% $C_8$, 12.0% $C_{10}$, 2.5% $C_{12}$ and 2% $C_{14}$. | 12.50 |

| | |
|---|---|
| "Cardura" E Ester (glycidyl ester of the formula 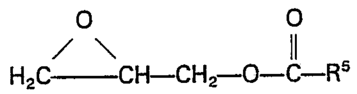 where $R^5$ is a tertiary aliphatic hydrocarbon group of 8—10 carbon atoms) | 12.50 |
| Titanium dioxide pigment | 125.00 |
| Magnesium Oxide (99.6% pure) | 12.50 |
| Blanc Fixe Pigment (Barium sulfate) | 112.50 |
| Methyl ethyl ketone | 18.44 |
| Butyl acetate | 18.44 |
| <u>Portion 2</u> | |
| Bicyclic amidine solution (10% solids in isopropanol of 1,8-diaza-bicyclo(5,4,0)undecene-7) | 2.50 |
| Curing agent F (prepared in Example 2) | 25.00 |
| Isopropanol | 22.50 |
| Total | 1000.00 |

Portion 1 is charged into a pebble mill and ground to a 0.5 mil fineness and charged into a mixing vessel and then portion 2 is added and mixed with portion 1 for to form a coating composition.

The resulting composition was reduced with methyl ethyl ketone solvent to a spray viscosity of about 37 seconds using a No. 2 Zahn cup and sprayed onto grit blasted steel panels and aluminum panels. In each case, thick films were applied by allowing flash drying between each application and the coatings were dried at ambient temperature in 24 hours to a tack free condition to provide a 1000 micron thick dry coating. In each case, the coatings had excellent adhesion to the metal substrate. After 7 days, the coatings were fully cured and were resistant to sulfuric acid, sodium hydroxide and solvents such as methyl ethyl ketone and ethyl acetate.

Example 9

Coating compositions 1—10 were prepared using the constituents shown in the attached Table. For each composition, the constituents of portion 1 were charged into a mixing vessel and thoroughly blended and portion 2 was added and blended. The resulting composition was reduced with methyl ethyl ketone solvent to a spray viscosity of about 25 seconds using a No. 2 Zahn cup and sprayed onto grit blasted steel panels and aluminum panels. In each case, thick films were applied by allowing flash drying between each application and the coatings were dried at ambient temperatures. The resulting dry coatings were 1000 microns thick. In each case, the coatings from compositions 1—10 had excellent adhesion to the metal substrate. After 7 days, each of the coatings of compositions 1—10 were fully cured and were resistant to sulfuric acid, sodium hydroxide and solvents such as methyl ethyl ketone and ethyl acetate.

TABLE

| Coating Composition | 1 | 2 | 3 | 4 | 5 (Parts By Weight) | 6 | 7 | 8 | 9 | 10 |
|---|---|---|---|---|---|---|---|---|---|---|
| Portion 1<br>Titanium dioxide pigment | 100 | ——→ | | | | | | | | |
| Magnesium oxide | 15 | ——→ | | | | | | | | |
| Fluorocarbon polymer solution (Described in Example 1 polymer having weight average molecular weight of 100,000) | 151.5 | 151.5 | 151.5 | 151.5 | 151.5 | — | — | — | — | — |
| Fluorocarbon polymer solutions (Same as Example 1 except polymer has a weight average molecular weight of 200,000) | — | — | — | — | — | 151.5 | 151.5 | 151.5 | 151.5 | 151.5 |
| Low molecular weight fluorocarbon polymer (60% vinylidene fluoride/40% hexafluoropropylene having a weight average molecular weight of 6400) | 25 | ——→ | | | | | | | | |
| Tetrafluoroethylene/perfluoroalkoxy vinyl ether copolymer (97/1 ratio) | 25 | — | — | — | — | 25 | — | — | — | — |
| Polytetrafluoroethylene | — | 25 | — | — | — | — | 25 | — | — | — |
| Fluorinated ethylene/propylene polymer | — | — | 25 | — | — | — | — | 25 | — | — |
| Polyvinyl fluoride | — | — | — | 25 | — | — | — | — | 25 | — |
| Polyvinylidene fluoride | — | — | — | — | 25 | — | — | — | — | 25 |
| Ethyl acetate | 50 | ——→ | | | | | | | | |
| Portion 2<br>Amine curing agent (prepared in Example 1) | 5.5 | ——→ | | | | | | | | |
| Bicyclic amidine (Described in Example 4) | 0.1 | ——→ | | | | | | | | |

Example 10

Coating compositions 11—13 were prepared as follows:

| | Coating Composition 11 | Coating Composition 12 (Parts by Weight) | Coating Composition 13 |
|---|---|---|---|
| **Portion 1** | | | |
| Polytetrafluoroethylene Powder | 56.25 | — | — |
| Fluorinated ethylene/propylene powder | — | 56.25 | — |
| Polyvinylidene Fluoride Powder | — | — | 56.25 |
| Fluorocarbon polymer solution (33.3% solids in ethyl acetate of a copolymer of 60% vinylidene fluoride and 40% hexafluoropropylene having a weight average molecular weight of about 400,000) | 430.74 | 430.74 | 430.74 |
| Perfluoroalkyl propylene oxide of the formula 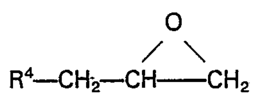 $R^4$ is a mixture of perfluoroalkyl group of 1% $C_4$, 48.5% $C_6$, 34.0% $C_8$, 12.0% $C_{10}$, 2.5% $C_{12}$ and 2% $C_{14}$. | 8.44 | 8.44 | 8.44 |
| "Cardura" E Ester (glycidyl ester of the formula 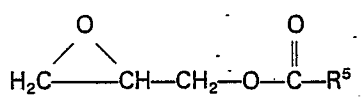 where $R^5$ is a tertiary aliphatic hydrocarbon group of 8—10 carbon atoms) | 8.44 | 8.44 | 8.44 |
| Titanium Dioxide Pigment | 213.75 | 213.75 | 213.75 |
| Magnesium Oxide (99.6% pure) | 8.44 | 8.44 | 8.44 |
| Butyl acetate | 112.21 | 112.21 | 112.21 |
| Methyl ethyl ketone | 111.73 | 111.73 | 111.73 |
| **Portion 2** | | | |
| Bicyclic amidine solution (10% solids in isopropanol of bicyclic amidine described in Example 4) | 16.87 | 16.87 | 16.87 |
| Amine Curing Agent (prepared in Example 1) | 15.35 | 15.35 | 15.35 |
| Isopropanol | 17.78 | 17.78 | 17.78 |
| Total | 1000.00 | 1000.00 | 1000.00 |

For each coating composition Portion 1 was charged into a pebble mill and ground to a fineness of 0.5 mils. Portion 2 was then mixed with Portion 1 to form a coating composition. The resulting composition was reduced with methyl ethyl ketone to a spray viscosity of about 25 seconds using a No. 2 Zahn cup and sprayed onto grit blasted steel panels and aluminum panels. In each case, thick films were applied by allowing flash drying between each application and the coatings were dried at ambient temperatures. The resulting dry coatings were a 1000 microns thick. In each case, the coatings from compositions 11—13 had excellent adhesion to the metal substrate. After 7 days, each of the coatings of composition 11—13 were fully cured and were resistant to sulfuric acid, sodium hydroxide and solvents such as methyl ethyl ketone and ethyl acetate.

## Claims

1. A curing agent for fluorocarbon polymers selected from the group consisting of

(1)

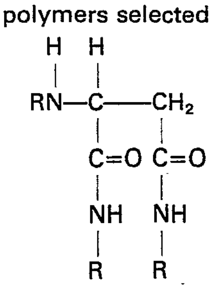

(2)

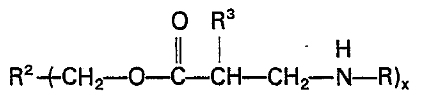

wherein R is $R^1NH_2$, $R^1$ is an aliphatic or cycloaliphatic hydrocarbon radical, $R^2$ is C or a hydrocarbon group, $R^3$ is H or $CH_3$, where x is 3 when $R^2$ is a hydrocarbon group and x is 4 when $R^2$ is C.

2. The curing agent of claim 1 in which $R^1$ is $-(CH_2)_6-$.

3. The curing agent of claim 1 having the formula:

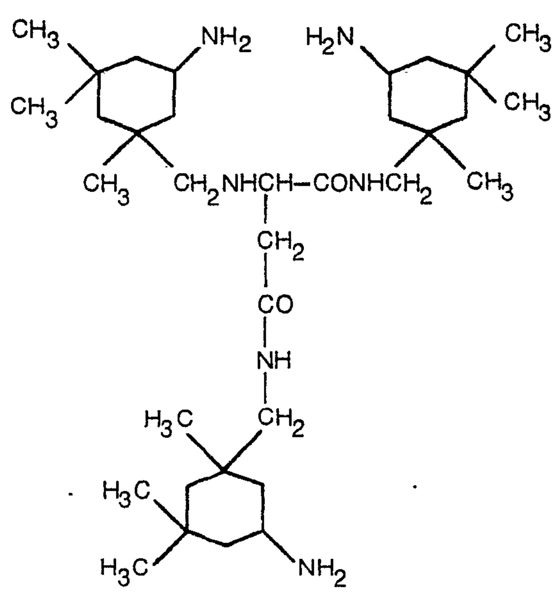

4. The curing agent of claim 1 having the formula

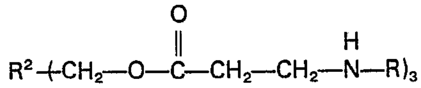

where R is a cycloaliphatic radical and $R^2$ is a hydrocarbon group.

5. The curing agent of claim 1 having the formula

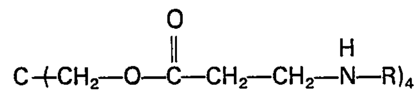

where R is cycloaliphatic radical.

6. The curing agent of claim 4 or claim 5 in which R is

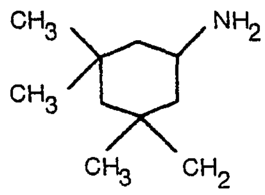

7. A coating composition containing a binder of a fluorocarbon polymer, a metallic oxide which is an acid acceptor and a curing agent as claimed in any preceding claim.

8. The composition of claim 7 containing about 0.01—3 percent by weight, based on the weight of the binder, of a bicyclic amidine.

9. The composition of claim 8 in which the bicyclic amidine is 1,8-diaza-bicyclo(5,4,0)undecene-7.

10. A composition as claimed in any one of claims 7 to 9 and comprising about 10—70% by weight binder and 30—90% by weight of an organic solvent; wherein the fluorocarbon polymer in the binder comprises vinylidene fluoride and hexafluoropropylene and has a weight average molecular weight of about 50,000—600,000.

11. The composition of claim 10 containing in addition about 1—20% by weight, based on the weight of the binder, of a perfluoroalkylpropylene oxide.

12. The composition of claim 11 in which the perfluoroalkylpropylene oxide has the formula

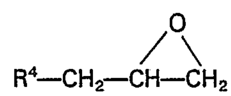

where $R^4$ is a perfluoroalkyl group having 4—12 carbon atoms.

13. The composition of any one of claims 10 to 12, containing in addition about 1—20% by weight, based on the weight of the binder, of a glycidyl ester of the formula

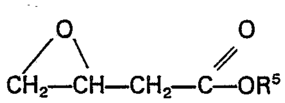

where $R^5$ is a tertiary aliphatic hydrocarbon group having 8—10 carbon atoms.

14. A composition as claimed in any one of claims 10 to 13 wherein the composition is a two-component composition where a first component consisting of the metallic oxide and a solution of the fluorocarbon polymer is mixed with the curing agent.

15. A coating composition as claimed in any one of claims 10 to 14 wherein the binder comprises a fluorocarbon polymer in solution comprising vinylidene fluoride and hexafluoropropylene and having a weight average molecular weight of about 5,000—600,000, dispersed fluorocarbon polymer particles having a particle size of about 1—150 microns and selected from polytetrafluoroethylene, fluorinated ethylene/propylene polymer, polyvinyl fluoride, polyvinylidene fluoride and a copolymer of tetrafluoroethylene/perfluoroalkoxyvinylether; a metallic oxide which is an acid acceptor; and a curing agent of either of the formulae (1) and (2) set forth and defined in claim 1, and wherein the organic solvent is a nonaqueous organic liquid.

16. The coating composition of claim 15 in which the binder comprises:

(a) 50—84.5% by weight of a fluorocarbon polymer in solution consisting essentially of polymerized units of 50—70% by weight, based on the weight of the polymer, of vinylidene fluoride and 30—50% by weight based on the weight of the polymer, of hexafluoropropylene and has a weight average molecular weight of about 75,000—450,000.

(b) 5—20% by weight of a low molecular weight fluorocarbon polymer in solution consisting essentially of polymerized units of 50—70% by weight, based on the weight of the polymer, of vinylidene fluoride and 30—50% by weight, based on the weight of the polymer, of hexafluoropropylene and has a weight average molecular weight of about 5,000—15,000;

(c) 5—20% by weight of dispersed fluorocarbon polymer selected from polytetrafluoroethylene fluorinated ethylene/propylene polymer, polyvinylfluoride, polyvinylidene fluoride and copolymer of tetrafluoroethylene/perfluoroalkoxyvinylether.

(d) 5—20% by weight, based on the weight of the binder, of magnesium oxide; and

(e) 0.5—20% by weight, based on the weight of the binder, of either (i) a curing agent of the formula:—

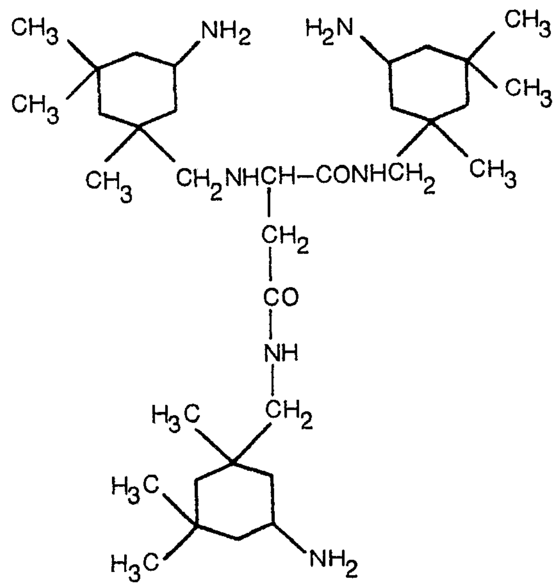

or (ii) a curing agent of the formula (2) set forth in claim 1, wherein $R^2$ is C or $C_3H_5$, $R^3$ is H and R is

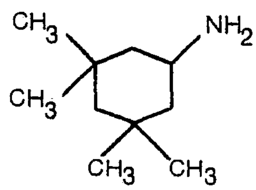

17. The coating composition of claim 16 containing titanium dioxide pigment in a pigment to binder weight ratio of about 20:100—200:100.

18. The coating composition of any preceding claim containing about 0.1—3% by weight of binder based on the weight of a bicyclic amidine.

19. A coating composition comprising about 10—70% by weight binder and 30—90% by weight of a nonaqueous organic liquid; wherein the binder comprises:

a fluorocarbon polymer in solution comprising vinylidene fluoride and hexafluoropropylene having a weight average molecular weight of about 5,000—600,000;

dispersed fluorocarbon polymer particles having a particle size of about 1—150 microns and being selected from polytetrafluoroethylene, fluorinated ethylene/propylene polymer, polyvinyl fluoride, polyvinylidene fluoride, and copolymer of tetrafluoroethylene/perfluoroalkoxy vinyl ether;

a curing agent of the formula:

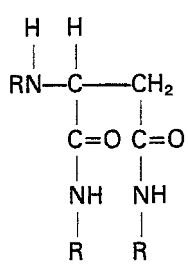

where R is $R^1NH_2$ and $R^1$ is aliphatic or cycloaliphatic hydrocarbon radical;

perfluoroalkylpropylene oxide; and a bicyclic amidine.

20. A coating composition comprising about 10—70% by weight binder and 30—90% by weight of a nonaqueous organic liquid; wherein the binder comprises:

a fluorocarbon polymer in solution comprising vinylidene fluoride and hexafluoropropylene having a weight average molecular weight of about 5,000—600,000,

dispersed fluorocarbon polymer particles having a particle size of about 1—150 microns and being selected from the group consisting of polytetrafluoroethylene, fluorinated ethylene/propylene polymer, polyvinyl fluoride, polyvinylidene fluoride, copolymer of tetrafluoroethylene/perfluoroalkoxyvinyl ether;

a curing agent of the formula:

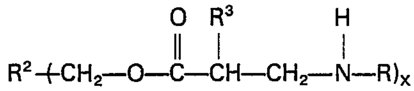

where R is a cycloaliphatic hydrocarbon radical, $R^3$ is H or $CH_3$, X is 3 when $R^2$ is a hydrocarbon group and X is 4 when $R^2$ is C;

perfluoroalkylpropylene oxide; and a bicyclic amidine.

21. A composition as claimed in any preceding claim containing about 5—15% by weight, based on the weight of the binder, of perfluoroalkylpropylene oxide.

22. A compound comprising

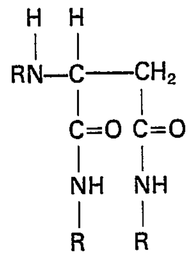

wherein R is $R^1NH_2$, $R^1$ is a cycloaliphatic hydrocarbon radical.

23. A compound comprising

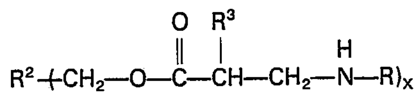

wherein R is $R^1NH_2$, $R^1$ is an aliphatic or cycloaliphatic hydrocarbon radical, $R^2$ is C or hydrocarbon group, $R^3$ is H or $CH_3$, where X is 3 when $R^2$ is a hydrocarbon group and X is 4 when $R^2$ is C.

**Patentansprüche**

1. Härtungsmittel für Fluorkohlenstoffpolymere, ausgewählt aus der Gruppe bestehend aus

(1)

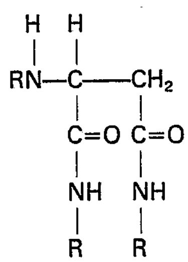

(2)

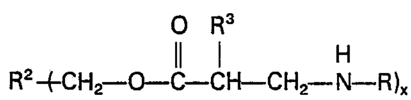

worin R $R^1NH_2$ ist, $R^1$ ein aliphatisches oder cycloaliphatisches Kohlenwasserstoffradikal ist, $R^2$ C oder eine Kohlenwasserstoffgruppe ist, $R^3$ H oder $CH_3$ ist, wobei x 3 ist, wenn $R^2$ eine Kohlenwasserstoffgruppe ist und x 4 ist, wenn $R^2$ C ist.

2. Härtungsmittel nach Anspruch 1, worin $R^1$ $(—CH_2—)_6$ ist.

3. Härtungsmittel nach Anspruch 1 mit der Formel

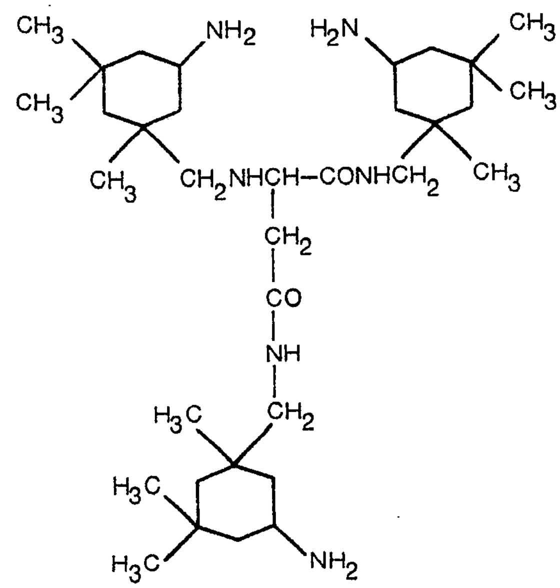

4. Härtungsmittel nach Anspruch 1 mit der Formel

$$R^2\text{-}(CH_2-O-\overset{\overset{O}{\|}}{C}-CH_2-CH_2-\overset{H}{N}-R)_3$$

worin R ein cycloaliphatisches Radikal ist und $R^2$ eine Kohlenwasserstoffgruppe ist.

5. Härtungsmittel nach Anspruch 1 mit der Formel

$$C\text{-}(CH_2-O-\overset{\overset{O}{\|}}{C}-CH_2-CH_2-\overset{H}{N}-R)_4$$

worin R ein cycloaliphatisches Radikal ist.

6. Härtungsmittel nach Anspruch 4 oder Anspruch 5, worin R

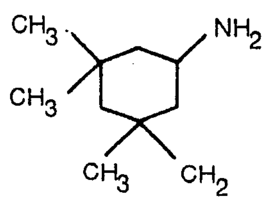

ist.

7. Beschichtungszusammensetzung, enthaltend einen Binder eines Fluorkohlenstoffpolymeren, ein metallisches Oxid, welches ein Säureakzeptor ist, und ein Härtungsmittel, wie es in einem der vorstehenden Ansprüche beansprucht ist.

8. Zusammensetzung nach Anspruch 7, enthaltend etwa 0,01 bis 3 Gew.-%, bezogen auf das Gewicht des Binders, eines bicyclischen Amidins.

9. Zusammensetzung nach Anspruch 8, worin das bicyclische Amidin 1,8-Diazabicyclo(5,4,0)undecen-7 ist.

10. Zusammensetzung nach einem der Ansprüche 7 bis 9 und enthaltend etwa 10 bis 70 Gew.-% Binder und 30 bis 90 Gew.-% eines organischen Lösungsmittels; worin das Fluorkohlenstoffpolymere im Binder Vinylidenfluorid und Hexafluorpropylen umfasst und ein Molekulargewicht nach dem Gewichtsmittel von etwa 50 000 bis 600 000 aufweist.

11. Zusammensetzung nach Anspruch 10, enthaltend zusätzlich etwa 1 bis 20 Gew.-%, bezogen auf das Gewicht des Binders, eines Perfluoralkylpropylenoxids.

12. Zusammensetzung nach Anspruch 11, worin das Perfluoralkylpropylenoxid die Formel

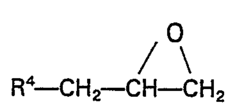

aufweist, worin $R^4$ eine Perfluoralkylgruppe mit 4 bis 12 Kohlenstoffatomen ist.

13. Zusammensetzung nach einem der Ansprüche 10 bis 12, enthaltend zusätzlich etwa 1 bis 20 Gew.-%, bezogen auf das Gewicht des Binders, eines Glycidylesters der Formel

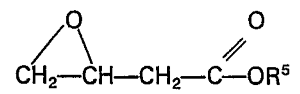

worin $R^5$ eine tertiäre aliphatische Kohlenwasserstoffgruppe mit 8 bis 10 Kohlenstoffatomen ist.

14. Zusammensetzung nach einem der Ansprüche 10 bis 13, worin die Zusammensetzung eine Zweikomponentenzusammensetzung ist, worin eine aus dem metallischen Oxid und einer Lösung des Fluorkohlenstoffpolymeren bestehende erste Komponente mit dem Härtungsmittel gemischt wird.

15. Beschichtungszusammensetzung nach einem der Ansprüche 10 bis 14, worin der Binder ein Fluorkohlenstoffpolymer in Lösung, welches Vinylidenfluorid und Hexafluorpropylen enthält und ein Molekulargewicht nach dem Gewichtsmittel von etwa 5 000 bis 600 000 aufweist, dispergierte Fluorkohlenstoffpolymerteilchen mit einer Teilchengrösse von etwa 1 bis 150 Mikron und ausgewählt aus Polytetrafluorethylen, fluoriertem Ethylen/Propylen-Polymer, Polyvinylfluorid, Polyvinylidenfluorid und einem Copolymeren von Tetrafluorethylen/Perfluoralkoxyvinylether; ein metallisches Oxid, welches ein Säureakzeptor ist; und ein Härtungsmittel nach jeder der in Anspruch 1 aufgeführten und definierten Formeln (1) und (2) umfasst und worin das organische Lösungsmittel eine nicht-wässrige organische Flüssigkeit ist.

16. Beschichtungszusammensetzung nach Anspruch 15, worin der Binder umfasst:

(a) 50 bis 84,5 Gew.-% eines Fluorkohlenstoffpolymeren in Lösung, das im wesentlichen aus polymerisierten Einheiten mit 50 bis 70 Gew.-%, bezogen auf das Gewicht des Polymeren, Vinylidenfluorid und 30 bis 50 Gew.-%, bezogen auf das Gewicht des Polymeren, Hexafluorpropylen enthält und ein Molekulargewicht nach dem Gewichtsmittel von etwa 75 000 bis 450 000 aufweist,

(b) 5 bis 20 Gew.-% eines Fluorkohlenstoffpolymeren niedrigen Molekulargewichts in Lösung, das im wesentlichen aus polymerisierten Einheiten mit 50 bis 70 Gew.-%, bezogen auf das Gewicht des Polymeren, Vinylidenfluorid und 30 bis 50 Gew.-%, bezogen auf das Gewicht des Polymeren, Hexafluorpropylen enthält und ein Molekulargewicht nach dem Gewichtsmittel von etwa 5 000 bis 15 000 aufweist;

(c) 5 bis 20 Gew.-% dispergiertes Fluorkohlenstoffpolymeres, ausgewählt aus Polytetrafluorethylen, fluoriertem Ethylen/Propylen-Polymer, Polyvinylfluorid, Polyvinylidenfluorid und Copolymerem aus Tetrafluorethylen/Perfluoralkoxyvinylether;

(d) 5 bis 20 Gew.-%, bezogen auf das Gewicht des Binders, Magnesiumoxid; und

(e) 0,5 bis 20 Gew.-%, bezogen auf das Gewicht des Binders, entweder (i) eines Härtungsmittels der Formel

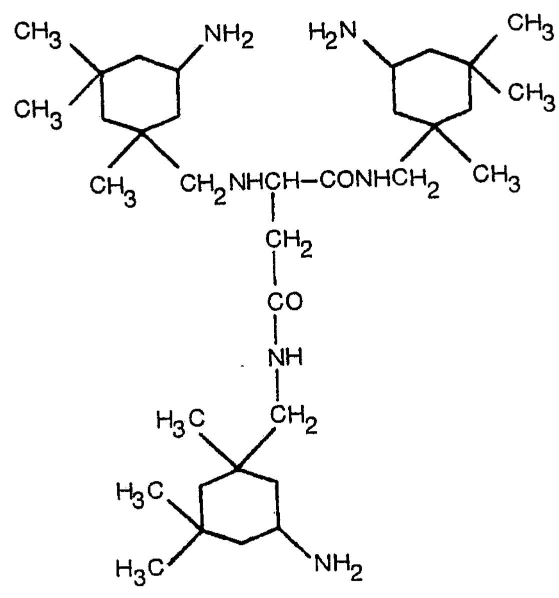

oder (ii) eines Härtungsmittels der in Anspruch 1 aufgeführten Formel (2), worin $R^2$ C oder $C_3H_5$ ist, $R^3$ H ist und R

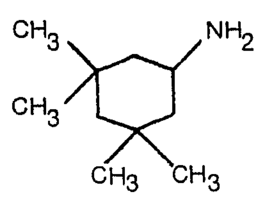

ist.

17. Beschichtungszusammensetzung nach Anspruch 16, enthaltend Titandioxidpigment in einem Pigment-zu-Binder-Gewichtsverhältnis von etwa 20:100 bis 200:100.

18. Beschichtungszusammensetzung nach einem der vorstehenden Ansprüche, enthaltend etwa 0,1 bis 3 Gew.-% des Binders, bezogen auf das Gewicht, eines bicyclischen Amidins.

19. Beschichtungszusammensetzung, enthaltend etwa 10 bis 70 Gew.-% Binder und 30 bis 90 Gew.-% einer nichtwässrigen organischen Flüssigkeit, worin der Binder umfasst:

ein Fluorkohlenstoffpolymeres in Lösung, welches Vinylidenfluorid und Hexafluorpropylen enthält und ein Molekulargewicht nach dem Gewichtsmittel von etwa 5 000 bis 600 000 aufweist;

dispergierte Fluorkohlenstoffpolymerteilchen, welche eine Teilchengröße von etwa 1 bis 150 Mikron aufweisen und ausgewählt sind aus Polytetrafluorethylen, fluoriertem Ethylen/Propylen-Polymer, Polyvinylfluorid, Polyvinylidenfluorid und Copolymer von Tetrafluorethylen/Perfluoralkoxyvinylether;

ein Härtungsmittel der Formel

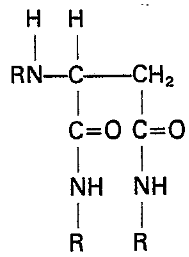

worin R $R^1NH_2$ ist und $R^1$ ein aliphatisches oder cycloaliphatisches Kohlenwasserstoffradikal ist;

Perfluoralkylpropylenoxid; und

ein bicyclisches Amidin.

20. Beschichtungszusammensetzung, enthaltend etwa 10 bis 70 Gew.-% Binder und 30 bis 90 Gew.-% einer nichtwässrigen organischen Flüssigkeit; worin der Binder umfasst:

ein Fluorkohlenstoffpolymer in Lösung, welches Vinylidenfluorid und Hexafluorpropylen enthält und ein Molekulargewicht nach dem Gewichtsmittel von etwa 5 000 bis 600 000 aufweist;

dispergierte Fluorkohlenstoffpolymerteilchen, welche eine Teilchengrösse von etwa 1 bis 150 Mikron aufweisen und ausgewählt sind aus der Gruppe, bestehend aus Polytetrafluorethylen, fluoriertem Ethylen/Propylen-Polymer, Polyvinylfluorid, Polyvinylidenfluorid, Copolymerem von Tetrafluorethylen/Perfluoralkoxyethylether;

ein Härtungsmittel der Formel

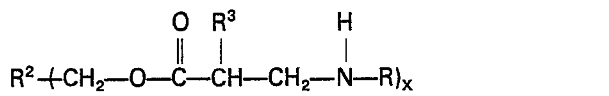

worin R ein cycloaliphatisches Kohlenwasserstoffradikal ist, $R^3$ H oder $CH_3$ ist, X 3 ist, wenn $R^2$ eine Kohlenwasserstoffgruppe ist, und X 4 ist, wenn $R^2$ C ist;

Perfluoralkylpropylenoxid; und

ein bicyclisches Amidin.

21. Zusammensetzung nach einem der vorstehenden Ansprüche, enthaltend etwa 5 bis 15 Gew.-%, bezogen auf das Gewicht des Binders, Perfluoralkylpropylenoxid.

22. Verbindung, enthaltend

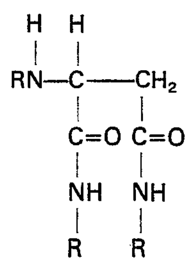

worin R $R^1NH_2$ ist, $R^1$ ein cycloaliphatisches Kohlenwasserstoffradikal ist.

23. Verbindung, enthaltend

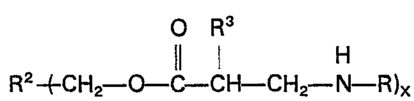

worin R $R^1NH_2$ ist, $R^1$ ein aliphatisches oder cycloaliphatisches Kohlenwasserstoffradikal ist, $R^2$ C oder eine

Kohlenwasserstoffgruppe ist, $R^3$ H oder $CH_3$ ist, wobei X 3 ist, wenn $R^2$ eine Kohlenwasserstoffgruppe ist, und X 4 ist, wenn $R^2$ C ist.

**Revendications**

1. Un agent de durcissement pour polymères fluorocarbonés, choisi dans le groupe formé par

(1)

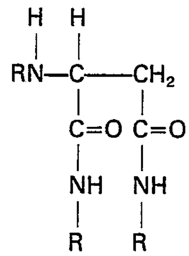

(2)

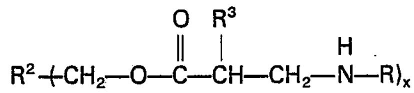

où R est $R^1NH_2$, $R^1$ est un radical hydrocarboné aliphatique ou cycloaliphatique, $R^2$ est C ou un groupe hydrocarboné, $R^3$ est H ou $CH_3$, et où x est 3 lorsque $R^2$ est un groupe hydrocarboné et x est 4 lorsque $R^2$ est C.

2. L'agent de durcissement de la revendication 1, dans lequel $R^1$ est $-(CH_2)_6-$.

3. L'agent de durcissement de la revendication 1, répondant à la formule:

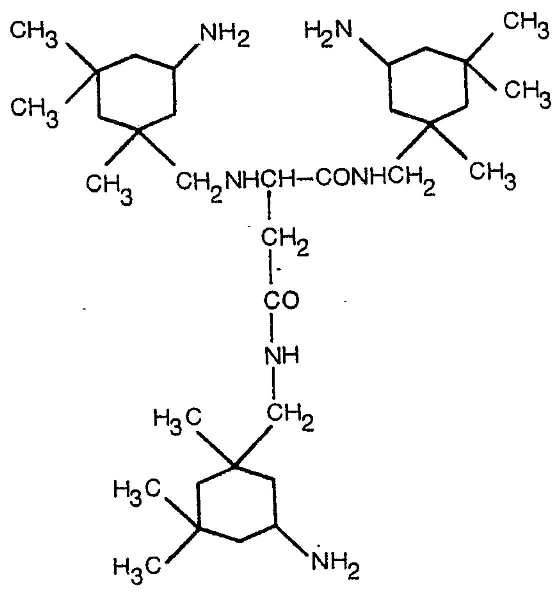

4. L'agent de durcissement de la revendication 1, répondant à la formule

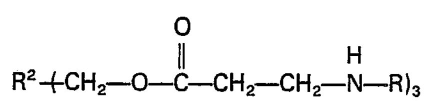

où R est un radical cycloaliphatique et $R^2$ est un groupe hydrocarboné.

5. L'agent de durcissement de la revendication 1, répondant à la formule

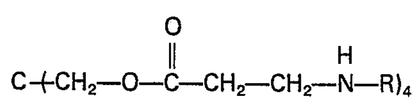

où R est un radical cycloaliphatique.

6. L'agent de durcissement de la revendication 4 ou la revendication 5 dans lequel R est

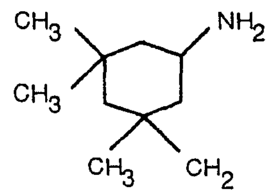

7. Une composition de revêtement contenant un liant constitué d'un polymère fluorocarboné, d'un oxyde métallique qui est un accepteur d'acide et d'un agent de durcissement tel que revendiqué dans l'une quelconque des revendications précédentes.

8. La composition de la revendication 7, contenant environ 0,01—3 pour cent en poids, par rapport au poids du liant, d'une amidine bicyclique.

9. La composition de la revendication 8, dans laquelle l'amidine bicyclique est le 1,8-diazabicyclo(5,4,0)undécène-7.

10. Une composition telle que revendiquée dans l'une quelconque des revendications 7 à 9 et comprenant environ 10—70% en poids de liant et 30—90% en poids d'un solvant organique; dans laquelle le polymère fluorocarboné du liant se compose de fluorure de vinylidène et d'hexafluoropropylène et a un poids moléculaire moyen en poids d'environ 50 000—600 000.

11. La composition de la revendication 10, contenant de plus environ 1—20% en poids, par rapport au poids du liant, d'un perfluoroalkyl-(oxyde de propylène).

12. La composition de la revendication 11, dans laquelle le perfluoroalkyl-(oxyde de propylène) répond à la formule

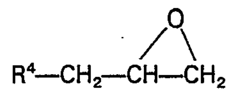

où $R^4$ est un groupe perfluoroalkyle ayant 4—12 atomes de carbone.

13. La composition de l'une quelconque des revendications 10 à 12, contenant de plus environ 1—20% en poids, par rapport au poids du liant, d'un ester glycidylique de la formule

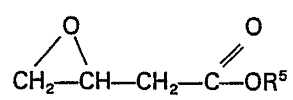

où $R^5$ est un groupe hydrocarboné aliphatique tertiaire ayant 8—10 atomes de carbone.

14. Une composition telle que revendiquée dans l'une quelconque des revendications 10 à 13, la composition étant une composition à deux composants où un premier composant constitué de l'oxyde métallique et d'une solution du polymère fluorocarboné est mélangé avec l'agent de durcissement.

15. Une composition de revêtement telle que revendiquée dans l'une quelconque des revendications 10 à 14, dans laquelle le liant comprend un polymère fluorocarboné en solution composé de fluorure de vinylidène et d'hexafluoropropylène et ayant un poids moléculaire moyen en poids d'environ 5000—600 000; des particules dispersées de polymère fluorocarboné ayant une grosseur de particules d'environ 1—150 micromètres et choisi parmi le polytétrafluoroéthylène, un polymère fluoré éthylène/propylène, le fluorure de polyvinyle, le fluorure de polyvinylidène et un copolymère tétrafluoroéthylène/éther perfluoroalcoxy vinylique; un oxyde métallique qui est un accepteur d'acide; et un agent de durcissement répondant à l'une ou l'autre des formules (1) et (2) exposées et définies dans la revendication 1, et dans laquelle le solvant organique est un liquide organique non aqueux.

16. La composition de revêtement de la revendication 15, dans laquelle le liant comprend:

(a) 50—84,5% en poids d'un polymère fluorocarboné en solution essentiellement constitué de motifs polymérisés de fluorure de vinylidène à raison de 50—70% en poids par rapport au poids du polymère, et d'hexafluoropropylène à raison de 30—50% en poids par rapport au poids du polymère, et ayant un poids moléculaire moyen en poids d'environ 75 000—450 000;

(b) 5—20% en poids d'un polymère fluorocarboné de bas poids moléculaire en solution essentiellement constitué de motifs polymérisés de fluorure de vinylidène à raison de 50—70% en poids par rapport au poids du polymère, et d'hexafluoropropylène à raison de 30—50% en poids par rapport au poids du polymère, et ayant un poids moléculaire moyen en poids d'environ 5 000—15 000;

(c) 5—20% en poids d'un polymère fluorocarboné dispersé choisi parmi le polytétrafluoréthylène, un polymère fluoré éthylène/propylène, le fluorure de polyvinyle, le fluorure de polyvinylidène et un copolymère tétrafluoroéthylène/éther perfluoroalcoxy vinylique;

(d) 5—20% en poids par rapport au poids du liant, d'oxyde de magnésium; et

(e) 0,5—20% en poids par rapport au poids du liant, de soit (i) un agent de durcissement de la formule:

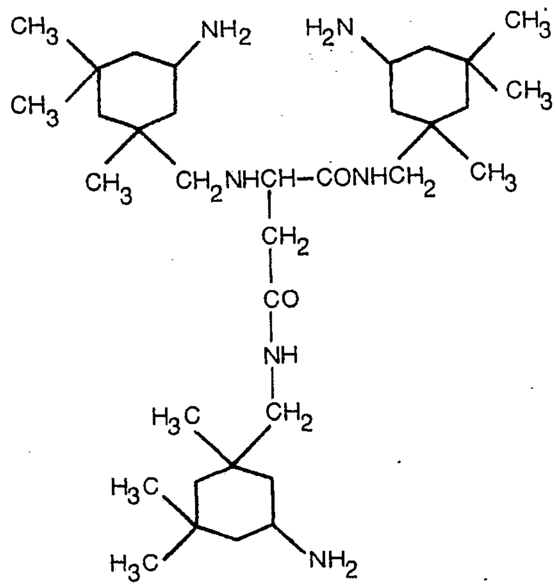

soit (ii) un agent de durcissement répondant à la formule (2) exposée dans la revendication 1, dans laquelle $R^2$ est C ou $C_3H_5$, $R^3$ est H et R est

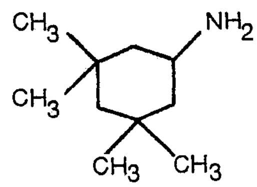

17. La composition de revêtement de la revendication 16, contenant du bioxyde de titane comme pigment en un rapport pondéral du pigment au liant d'environ 20:100—200:100.

18. La composition de revêtement de l'une quelconque des revendications précédentes, contenant environ 0,1—3% en poids, par rapport au poids du liant, d'une amidine bicyclique.

19. Une composition de revêtement comprenant environ 10—70% en poids de liant et 30—90% en poids d'un liquide organique non aqueux; dans laquelle le liant comprend:

un polymère fluorocarboné en solution composé de fluorure de vinylidène et d'hexafluoropropylène et dont le poids moléculaire moyen en poids est d'environ 5 000—600 000;

des particules dispersées de polymère fluorocarboné ayant une grosseur de particules d'environ 1—150 micromètres et qui est choisi parmi le polytétrafluoroéthylène, un polymère fluoré éthylène/propylène le fluorure de polyvinyle, le fluorure de polyvinylidène et un copolymère tétrafluoroéthylène/éther perfluoroalcoxy vinylique;

un agent de durcissement de la formule:

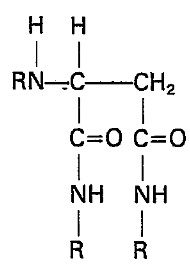

où R est $R^1NH_2$ et $R^1$ est un radical hydrocarboné aliphatique ou cycloaliphatique;

un perfluoroalkyl-(oxyde de propylène); et

une amidine bicyclique.

20. Une composition de revêtement comprenant environ 10—70% en poids de liant et 30—90% en poids d'un liquide organique non aqueux; dans laquelle le liant comprend:

un polymère fluorocarboné en solution composé de fluorure de vinylidène et d'hexafluoropropylène et dont le poids moléculaire moyen en poids est d'environ 5 000—600 000;

des particules dispersées de polymère fluorocarboné ayant une grosseur de particules d'environ 1—150 micromètres et qui est choisi dans le groupe formé par le polytétrafluoroéthylène, un polymère fluoré éthylène/propylène, le fluorure de polyvinyle, le fluorure de polyvinylidène, un copolymère tétrafluoroéthylène/éther perfluoroalcoxy vinylique;

un agent de durcissement de la formule:

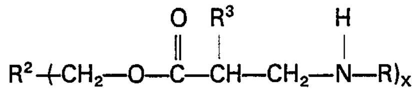

où R est un radical hydrocarboné cycloaliphatique, $R^3$ est H ou $CH_3$, X est 3 lorsque $R^2$ est un groupe hydrocarboné et X est 4 lorsque $R^2$ est C;

un perfluoroalkyl-(oxyde de propylène); et

une amidine bicyclique.

21. Une composition telle que revendiquée dans l'une quelconque des revendications précédentes, contenant 5—15% en poids par rapport au poids du liant, de perfluoroalkyl-(oxyde de propylène).

22. Un composé de la formule

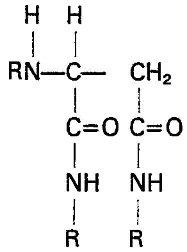

dans laquelle R est $R^1NH_2$, $R^1$ est un radical hydrocarboné cycloaliphatique.

23. Un composé de la formule

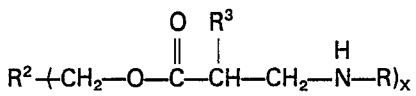

dans laquelle R est $R^1NH_2$, $R^1$ est un radical hydrocarboné aliphatique ou cycloaliphatique, $R^2$ est C ou un groupe hydrocarboné, $R^3$ est H ou $CH_3$, et où X est 3 lorsque $R^2$ est un groupe hydrocarboné et X est 4 lorsque $R^2$ est C.